# EUROPEAN PATENT APPLICATION

(11) **EP 3 498 090 A1**
(43) Date of publication of application: **19.06.2019**
(21) Application number: 18213165.6
(22) Date of filing: 17.12.2018
(51) Int. Cl.: A01K 15/02, A01K 29/00, A01K 27/00

(54) **AUTOMATIC ANIMAL POSTURE CONDITIONING SYSTEM**

(30) Priority: 15.12.2017 PT 2017110459
(71) Applicant: Ifarmtec - Intelligent Farm Technologies, LDA, 3680-264 São João Da Serra (PT)
(72) Inventor: Campos Bartolomeu, Paulo Jorge, 3770-220 Oliveira do Bairro (PT); Sousa Gonçalves, Pedro Alexandre, 3830-028 Ílhavo (PT); Reis Pedreiras, Paulo Bacelar, 3770-013 Bustos (PT); Quental Rio Gonçalves, Maria da Graça, 3830-028 Ílhavo (PT)
(74) Representative: Lourenço Martinho do Rosário, Ana Margarida

(57) **Abstract**

The present invention refers to an automatic animal posture conditioning system, comprising at least one mobile device (1) (2) fixed to the body of an animal by a suitable mechanical means and, depending on the embodiment, by one or more external devices (3). The mobile device (1) and the auxiliary mobile device (2) comprise a collection of means for collecting data which enable to identify the relative angular position between the various parts of the animal body, animal body oscillatory movements and animal body static accelerations. In addition, the mobile device (1) comprises a set of means for applying sensory stimuli to the animal, whenever the latter adopts an unwanted posture. In addition, the mobile device (1) includes a central data processing unit, which processes the data that it receives from the various components, which verifies whether there is an infraction or not, and if any, that defines what stimulus to be applied to the animal.

## Description

### Technical Field

The present invention belongs to the field of devices and methods for animal control, more specifically, it relates to a system comprising one or more devices and a method for automatic postural conditioning of animals, more specifically their cervicodorsal and/or lumbar posture, depending on the position in the body of the animal where the equipments are placed.

### Background of the Invention

One of the most common animal conditioning practices is their immobilization, a usual practice when animals suffer muscular orthopaedic accidents or for some reason suffer cutaneous wounds. The commonly used solution consists in the adoption of a form of movement restriction, such as the use of tight splints, immobilizing the part of the body in question. This type of practice is reasonably suitable for companion animals, but when applied to other animals such as cows, sheep and goats, it raises major hygiene issues, because of the environment in which they live, and because they spend a lot of time outdoors, exposed to rain, dust and to the sun.

In the case of non-orthopaedic surgeries or wounds, the animals tend to lick them, often leading to the use of *Elizabethan collars* as a way to protect the treatments and/or stitches that close the cutaneous incisions.

Another situation where animal confinement is necessary occurs in cases where they should be prevented from circulating in a particular space, for example because a chemical treatment has been applied in certain areas, or because it is intended to prevent animals from feeding on certain fruits or plants. In these cases, it is common to confine the animals to closed spaces with fences, depriving them unnecessarily of access to the outside environment, even if the area to be isolated is a relatively small plot compared to the whole pasture area.

Conversely, there is sometimes interest in defining specific regions for animals to move or feed. One of these cases are guard dogs, where it may be important to assure that they feed in a specific place, to prevent their accidental or intentional poisoning.

Or it may still be the case that animals need to pass through a certain area but cannot feed on it. Or that they can feed but only from food above a certain height.

Females' fertility, and the reproductive control of animals, requires surgeries, the administration of contraceptive drugs, or the closure of animals, with obvious drawbacks, such as side effects for the case of hormonal treatments, or the inconvenience of locking up the animals. In addition, when females are enclosed, episodes of invasion of the enclosure space by males are frequent, eventually resulting in accidents related to the unwanted conception. Although surgery is safer in terms of contraceptive control, and does not cause side effects due to hormonal components, it is irreversible and costly.

### Background of the Invention

A number of devices which enable monitoring and/or animal conditioning have been identified in the state of the art.

For example, document WO2013005213 discloses a system for monitoring the location of one or more animals.

Document EPO808567 discloses an apparatus for collecting and processing data for monitoring animal condition and activity.

Moreover, document EP2798948 presents a method that allows monitoring the animals' body behaviour, decoding their state of health and fertility.

Other documents refer to devices and methods for conditioning the behaviour of animals.

Namely, document US5794569 discloses a method and an apparatus for electronically confining an animal within one or more selected spaces within a designated area. This confinement is achieved through sensorial stimuli as a way to penalize the animal whenever it goes out of the designated area, and can take the form of an acoustic signal or an electrostatic stimulus. Other documents refer to equipment and/or methods with a similar purpose, as for example documented in US3753421, or in US4898120, or in US5067441.

A remark should also be made to document US5791294, which discloses an apparatus and method for monitoring and controlling the position of an animal by applying sensorial stimuli in order to motivate the animal to move.

Another type of animal conditioning is the control of bark of canids, and one of the possible methods to do it can be found in document US2009194037.

Document PT108116, which may be considered the closest state of the art, proposes a device based on a stimulus-emitting collar to oblige herbivorous animals to feed on ground weeds. Such mechanism, despite implementing a form of animal and food conditioning, is very limited in applicability because it is based only on ultrasound sensors that measure the distance from the neck of the animal to the ground. The use of ultrasound sensors is very prone to errors in irregular terrain, where there are stones or thicker plants such as flowers, and therefore not suitable for stimulating animal learning. On the other hand, measuring the distance from the neck to the ground is not sufficient to infer about the overall posture of the animal.

### Summary of the Invention

This invention presents a system comprising at least one device and a method for postural conditioning of animals, which is intended to restrict the type of movements and actions that they may adopt without being otherwise deprived of their freedom of movement.

The system herein described is implemented in the form of at least one self-powered mobile device fixed to the body of the animal by a suitable mechanical means, such as a necklace, a collar or a harness, applied to the cervicodorsal and/or lumbar area of the animal.

The postural conditioning of animals is done in an automated way, without requiring direct human intervention, but without impeding said intervention from occurring.

This invention is applicable to animals with a cognitive level that allows the identification of auditory, vibratory and electrostatic stimuli, such as bovine animals, sheep, goats, canids, among others.

The device implements a stimulus-based methodology that promotes the learning process through experience, where an action produces an automatic reflex (Pavlov reflex). Animals, through a repetitive learning process, recognize sound and vibratory stimuli, learning that they precede electrostatic stimuli, if they do not reverse their posture or action.

The device described in the present invention is equipped with a set of means for inferring the posture of an animal, including but not limited to, a set of sensors, namely ultrasonic, inertial and electrostatic, among others, that using suitable filtering and data, allowing to infer the posture of the animal in a more comprehensive and reliable way than using only a single sensor.

This way, different types of postures can be discriminated, as well as a significantly increase on the reliability of the detection is attained, which has a significant impact on the system efficiency and on the ease of the learning/training process of the animal.

In addition, the device is equipped with a set of means for applying on the animal a set of sensorial stimuli, namely but not limited to, sound, vibratory and electrostatic stimuli. The device begins by applying sound and vibratory stimuli, so that the animal associates the stimulus to the adopted posture and/or irregular action. If there is no reversion of the behaviour, the device then applies the electrostatic stimulus as a way to penalize the animal and thus induce the reversal of its behaviour.

The cadence and sequence of application of the aforementioned stimuli are defined by an algorithm that takes into account the history as well as the learning process of the animal. The frequency, duration and intensity of the stimuli are defined according to the species, gender and size of the animal.

The system also includes optional external units that, when present, allow to delimit the geographical regions where the postural conditioning is applied, as well as to define the type of postures that should be allowed and penalized in each one of these regions.

External units, as well as devices placed on animals, have a radio transceiver used for relative location and communication purposes. When configured for this mode of operation, the devices placed on the animals estimate the relative position between the animal and the external unit(s), using a suitable method, namely but not limited to, received signal strength, triangulation, etc.

The external units encode in the messages they send the types of behaviours to be penalized in their region of influence, thus allowing the system to adapt the decision algorithm to the place where the animal currently is.

The device placed on the animal may collect and transfer data and statistics relating to its own operating state (battery status, number of times that stimuli were applied, etc.). This is done through the aforementioned transceiver, being the data of each animal collected by one or more external units.

These external units have a network interface that allows downloading this data to a computer.

### Advantages of the invention

The subject of the invention is a solution that presents a novel and innovative method that aims to determine whether the conditions dictating the situations in which postural conditioning is applied and which stimulus to be applied, by determining the position of the body of the animal, is or is not being fulfilled.

### Brief description of drawings

These and other features can be easily understood from the accompanying drawings, which should be considered as mere examples and not in any way restrictive of the scope of the invention. In the drawings and for illustrative purposes, the measurements of some elements may be exaggerated and not drawn to scale. Absolute and relative dimensions do not correspond to the real ratios for carrying out the invention.

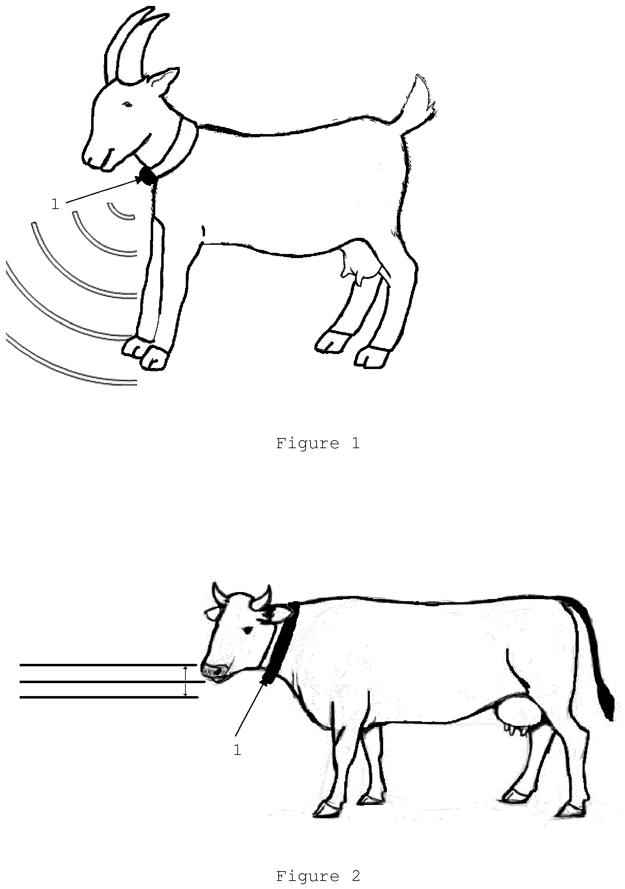

Figure 1 shows a schematic representation of the application of the collar on a goat and may also be used on other types of animals.
Figure 2 depicts the application of the device to a bovine, illustrating the operation of the device by immobilizing the cervical/lumbar zone.

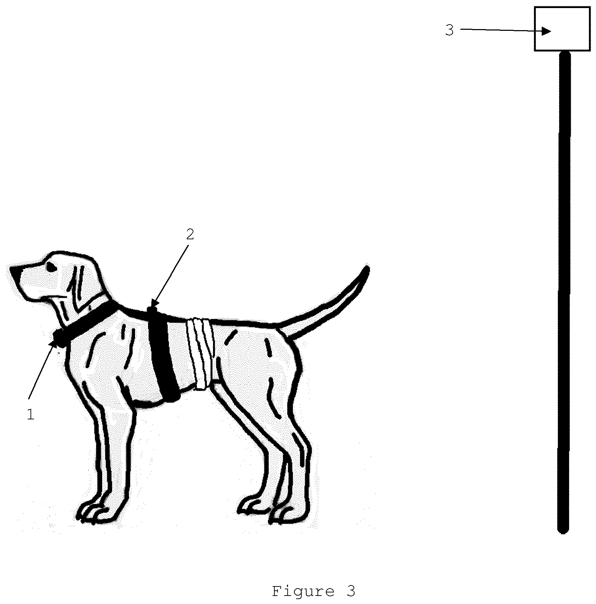

Figure 3 illustrates the operation of the system applied to a canine, demonstrating the effect of wound protection, using a mobile device (1) applied through a collar, an auxiliary mobile device (2) applied through a harness, and an external unit (3) applied on a pole.

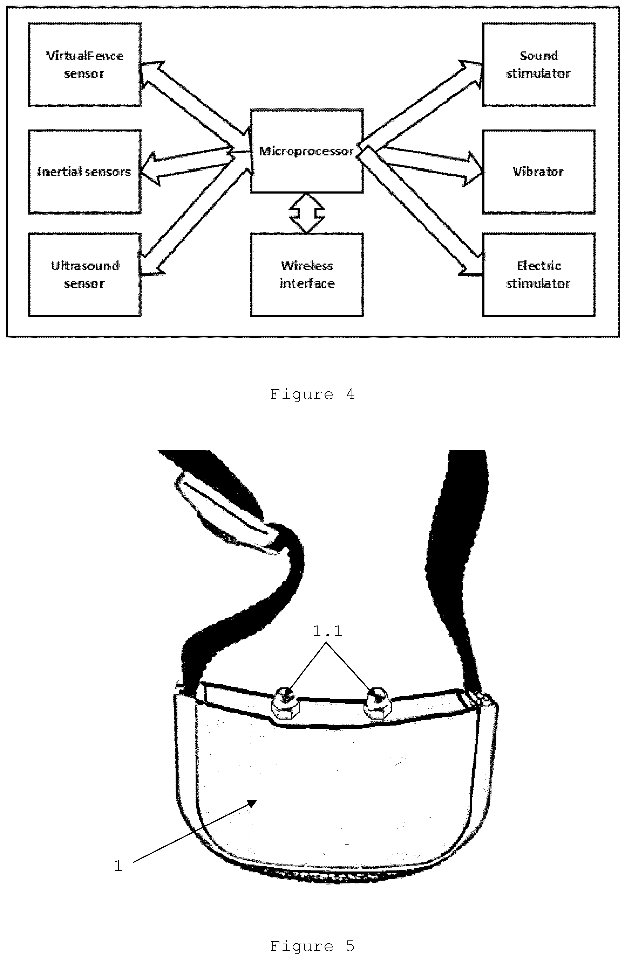

Figure 4 shows the block diagram illustrating the modules that compose the device, as well as the interaction of the components of the postural control system.
Figure 5 is a schematic and simplified perspective view of an embodiment of the necklace/collar supporting the mobile device (1) and the electrostatic stimulator emitting electrodes (1.1).
Figure 6 shows the sequence of operations of the animal posture control process.
Figure 7 shows the sequence of operations of one of the embodiments, more particularly of the "eating" behaviour.
Figure 8 shows the sequence of operations of another embodiment, more particularly of detecting situations in which the animal licks wounds or other types of lesions.

In figure 9 it is possible to observe a representation of the posture control in which the animal cannot eat below a certain level, being visible the trace that delimits the zones of non-infraction and infraction, the zones of non-infraction and of infraction, the mobile device (1) and the auxiliary mobile device (2).

In figure 10 it is possible to observe a representation of the posture in which the animal cannot eat above a certain level, being visible the trace that delimits the zones of non-infraction and infraction, the zones of non-infraction and of infraction, the mobile device (1) and the auxiliary mobile device (2).

In figure 11 it is possible to observe a representation of the posture in which the animal tries to lick wounds or other type of injuries.

In figure 12 it is possible to observe an animal posture representation that has to be maintained in case of injuries such as, for example, a neck injury.

In figure 13 it is possible to observe a representation of the posture that the animal presents in copulation situations.

### Detailed description of the Invention

The animal conditioning mechanism herein described is based on the classical/pavlovian conditioning process, with the modification of certain behaviours by means of the stimulus-response combination effects on the central nervous system of the animals. These learn to associate certain types of behaviours with stimuli or sequences of stimuli. The literature reports that the use of stimuli sequences, with increasing gradation in terms of discomfort, is more effective than the use of a single stimulus, which is why we have chosen to apply sequences of stimuli composed by sounds, vibrations and electrostatic discharges.

The system encompasses a set of devices that depend on the embodiment:
- a mobile device (1) applied to a collar, which allows controlling the cervicodorsal posture of animals when placed on the neck or back of the animal;
- an auxiliary mobile device (2) applied to a harness that can be placed on the back of the animal to monitor its lumbar posture;
- an external device (3), which receives information and statistics about the condition of the animal and the status of its devices via a communication system, and sends parameterization and command information to/from the mobile device (1) and the auxiliary mobile device (2).

The mobile device 1 and the auxiliary mobile device 2 incorporate a set of components that enable the collection of elements allowing ascertaining the animal posture, including, but not limited to, sensors, namely ultrasonic, inertial (accelerometers) and electrostatics, among others, and location sensors (e.g. based on RSSI or GPS). The mobile device (1) also incorporates a set of components that allow the application of stimuli to the animal, namely the application of sound, vibratory and electrostatic stimuli. The components that apply the stimuli are selected from the market available parts and that best fit the animal for which they are intended. In the case of electrostatic stimuli, the mobile device (1) is equipped with at least one pair of electrodes (1.1) that are in permanent contact with the skin of the animal.

In addition, the mobile device 1 includes a central data processing unit, which processes the received data from the various components and verifies whether there is an infraction or not, and, if any, what stimulus is to be applied to the animal. This central data unit may communicate with an external device (3) by any known method, with or without wires, in order to send and/or receive information, in particular regarding its operation, the condition of the animals or other relevant information that may require the intervention of a person in charge. The central unit can also receive various information such as, for example, the areas where the animal can move or feed, how high they can feed, if they can only eat grass and fruits that are on the ground, or if they can be fed from leaves that are in a higher position, or if they can go a little higher.

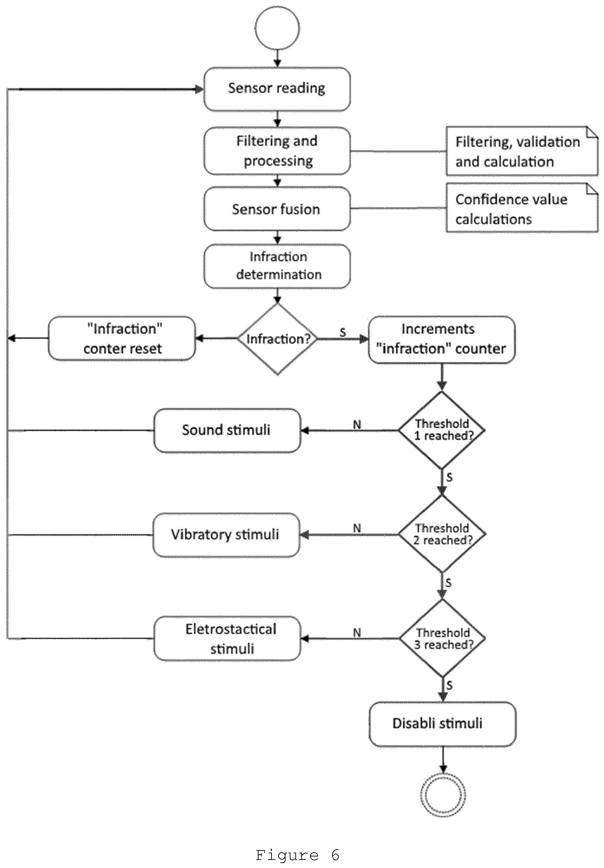

Generally, the posture control process is conducted in a sequence of operations that are executed cyclically as illustrated in Figure 6. In a first step, the sensors are read and a pre-processing is performed, comprising a set of operations well known in the art, such as validation, filtration and sensor fusion, in order to obtain a high degree of confidence in the readings carried out.

Next, the data is analysed in order to determine the existence of infringement situations. If the posture of the animal is correct, the cycle repeats itself. Conversely, if an infraction is detected, the stimuli sequence is applied. Initially, the initial stimulus, namely but not exclusively, a sound stimulus is applied. If the animal persists in the infringement behaviour, a first threshold is exceeded (threshold 1), leading to the application of the intermediate stimulus. If the animal persists in the infringement, a second threshold is reached (threshold 2), and the most severe stimulus (electrostatic discharge) is applied. At any time, a reversal in the animal behaviour interrupts the stimuli sequence. If the infringement behaviour is not interrupted within a certain limit, the stimulation ceases (threshold 3). This scenario predicts situations of hardware failure or poor animal learning, thus avoiding unnecessarily penalizing the animal.

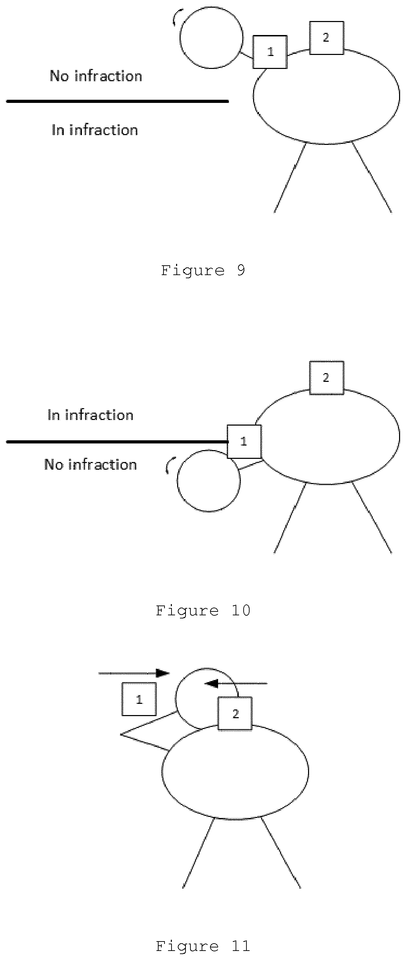

In one embodiment, the system is applied to the "eating" behaviour. This embodiment can be segmented into two distinct embodiments. A first embodiment in which the animal cannot eat below a certain level is documented in the posture shown in Figure 9. A second embodiment in which the animal cannot eat above a certain level is represented in Figure 10. This behaviour is identified by placing a mobile device (1) applied to a collar on the neck or back of the animal. Fundamentally, what is analysed in these embodiments is whether the animal is feeding within a previously defined area and if the height of the food being consumed is the intended one (food in the ground or above). Typically, these are scenarios occurring with goats and sheep graze all day in herds and without a fixed position to feed. Thus, one may want to avoid that animals feed in an area that has recently been treated and contains chemicals, invades land belonging to another owner or go to a road. The analysis of the height at which the animal is feeding is required in areas of land where, for example, besides grass, there are flowers, shrubs, trees, etc., such as vines and orchards, and where feeding from the ground is allowed (grass, flowers, leaves or fallen fruits), but feeding on flowers, leaves or fruits found in the bushes or in the trees is prohibited.

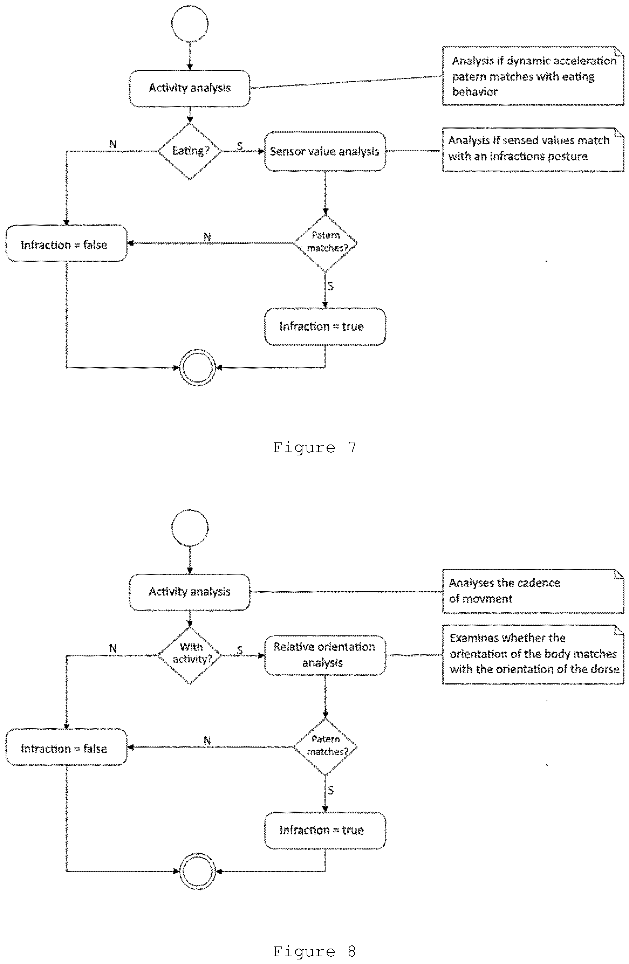

This embodiment is illustrated in figure 7. By analysing the dynamic acceleration provided by the accelerometer it is possible to infer if the animal is eating. The temporal variation of the dynamic acceleration measured by the accelerometer allows to discriminate certain types of behaviours. Some examples:
- if it is null the animal is still,
- if it has high peaks of acceleration, alternately positive and negative, on the vertical axis with a high frequency, the animal is running,
- if it has medium/low acceleration peaks, alternately positive and negative, on the vertical axis, with a low frequency, the animal is walking.

Through appropriate techniques (e.g., *machine learning*) and using dynamic acceleration, it is possible to identify behaviours such as rest, eating, running, walking, etc. When an eating behaviour is identified, an analysis of the associated pattern is conducted in order to evaluate if it is occurring within the permitted bounds. Several tests are possible. Based on its position and/or behaviour sensors (accelerometer and/or ultrasound) it is possible infer if the animal is eating from the ground (e.g., herbs) or from above (e.g., leaves or fruits). Using location sensors (e.g., based on RSSI or GPS) it is possible to know where the animal is. This information is used to compose analysis rules determining the existence of an infraction.

In a third embodiment, the system is directed to detecting situations in which the animal licks wounds or other types of injuries. This implementation is illustrated in Figure 8 and the posture is documented in Figure 11. This behaviour is identified primarily by placing a mobile device (1) applied to a collar on the neck of the animal, and an auxiliary mobile device (2) applied in a harness on the back of the animal. In a regular posture the relative angle between both devices is less than 90 degrees. When the animal turns back, the angular difference approaches 180 degrees. In this case, inertial sensors (e.g., accelerometer) are used to determine if the posture of the animal is natural (e.g., looking back to identify the presence of predators or other animals) or if it is an infraction. In this case, the animal lowers the head and produces movements that have a distinct signature, measurable through the dynamic acceleration of the neck.

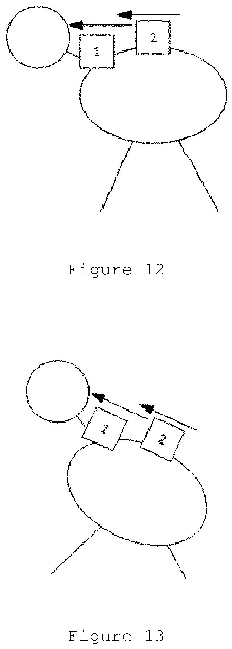

In a fourth embodiment, the system is directed to situations in which the animal needs to be immobilized due to injuries and/or surgeries, for example a neck injury, wherein the mobile device (1) and the mobile device (2) must be coplanar. This implementation is illustrated in Figure 12. This behaviour is identified primarily by placing a mobile device (1) applied to a collar on the neck of the animal, and an auxiliary mobile device (2) applied to a harness on the back of the animal. In a regular posture both devices make a certain angle. When the animal bends or turns, the angular difference detected by the two devices allows the unwanted posture detection.

In a fifth embodiment, the system is directed to the detection of copulation. This implementation is illustrated in Figure 13, which shows the example of a male, but can also be applied to females. This behaviour is identified primarily by placing a mobile device (1) applied to a collar on the neck of the animal, and an auxiliary mobile device (2) applied to a harness on the back of the animal. In this scenario, the dynamic accelerations measured by the inertial sensors exhibit specific patterns resulting from the oscillatory movement of the animal occurring during the intercourse practice. Furthermore, the static accelerations remain constant and allow ascertaining the inclination of the neck and the body of the animal. In this case, inertial sensors (e.g., accelerometer) are used to detect if the posture of the animal is natural (e.g., scratching) or it is an infraction (copulating). During copulation the body of the animal has a given inclination that can be detected by its static acceleration and depends on species, gender and size. Moreover, the oscillatory movement is characterized by a distinct signature that also varies with the gender, measurable through the dynamic acceleration of the neck.

## Claims

1. Automatic animal posture conditioning system comprising at least one mobile device (1) (2) equipped with means for inferring the posture and displacement of an animal, and means for applying to the animal sensory stimuli, **wherein** the means for inferring the animal posture and displacement collect proceed to the collection of elements that determine the angular position of various parts of the animal body with each other, oscillatory movements of the body of the animal and static accelerations of the body of the animal.

2. Automatic animal posture conditioning system according to claim 1, **wherein** the means for inferring the animal posture are sensors.

3. Automatic animal posture conditioning system according to claim 2 **wherein** the sensors are ultrasonic, inertial (accelerometers) electrostatic and location sensors.

4. Automatic animal posture conditioning system according to claim 3 **wherein** the location sensors are based on RSSI or GPS.

5. Automatic animal posture conditioning system according to claim 1 **wherein** the sensorial stimuli applied to the animal are sound, vibratory and electrostatic stimuli.

6. Automatic animal posture conditioning system according to claim 1 **wherein** the electrostatic stimuli are applied via a pair of electrodes (1.1) which are in contact with the skin of the animal.

7. Automatic animal posture conditioning system according to claim 1 **wherein** the mobile device (1) and the auxiliary mobile device (2) are fixed to body of the animal by a suitable mechanical means, applied to the cervicodorsal and/or lumbar area of the animal.

8. Automatic animal posture conditioning system according to the previous claims **wherein** the system being applicable to animals with a cognitive level that allows for the recognition of auditory, vibratory and electrostatic stimuli.

9. Automatic animal posture conditioning method of the system claimed in previous claims **wherein** comprises the following steps:
a) the system being parameterized according to the infraction that should be prevented, the species, the condition, the size and the postural history of the animal,
b) the sensors are read,
c) in order to obtain a high degree of confidence in the readings carried out, validation, filtration and sensorial fusion are carried out,
d) the posture of the animal being determined,
e) being verified if the posture of the animal is correct, determining whether there is an infraction,
f) if no infraction is detected, returns to step b),
g) the value of the "infraction" counter is incremented,
h) the value of the "infraction" counter is compared with the value of "threshold 1",
i) if the value of "threshold 1" is lower than the value of the "infraction" counter, the initial stimulus is applied and returns to step b),
j) the value of the "infraction" counter is compared with the value of "threshold 2",
k) if the value of "threshold 2" is lower than the value of the "infraction" counter, the intermediate stimulus is applied and returns to step b),
l) the value of the "infraction" counter is compared with the value of "threshold 3",
m) if the value of "threshold 3" is lower than the value of the "infraction" counter, the most severe stimulus is applied and returns to step b),
n) stimuli application ceases.

10. Method according to the previous claim **wherein** the initial stimulus to be a sound stimulus.

11. Method according to claim 9 **wherein** the intermediate stimulus to be a vibratory stimulus.

12. Method according to claim 9 **wherein** the most severe stimulus to be an electrostatic stimulus.
